# EUROPÄISCHE PATENTSCHRIFT

(11) **EP 1 368 491 B1**
(45) Veröffentlichungstag und Bekanntmachung des Hinweises auf die Patenterteilung: **10.09.2008**
(21) Anmeldenummer: 01986325.7
(22) Anmeldetag: 02.10.2001
(51) Int. Cl.: C12Q 1/68

(54) **VERFAHREN ZUR SELEKTION HOCHAFFIN AN EIN TARGET BINDENDER NUKLEINSÄUREN DURCH ZWEIDIMENSIONALE AUFTRENNUNG**
METHOD OF SELECTION, BY TWO-DIMENSIONAL SEPARATION, OF NUCLEIC ACIDS THAT BIND TO A TARGET WITH HIGH AFFINITY
PROCEDE POUR SELECTIONNER DES ACIDES NUCLEIQUES SE LIANT AVEC UNE GRANDE AFFINITE A UNE CIBLE, PAR SEPARATION EN DEUX DIMENSIONS

(30) Priorität: 03.10.2000 DE 10048944
(43) Veröffentlichungstag der Anmeldung: 10.12.2003
(73) Patentinhaber: AptaRes AG, 14943 Luckenwalde (DE)
(72) Erfinder: Kage, Andreas, Dr., 12007 Berlin (DE)
(74) Vertreter: Jungblut, Bernhard Jakob
(86) Internationale Anmeldenummer: PCT/DE2001/003818
(87) Internationale Veröffentlichungsnummer: WO 2002/029094

(56) Entgegenhaltungen:
- EP-A- 1 006 362
- DE-A- 10 049 074
- US-A- 5 707 796
- THIESEN H J ET AL: "TARGET DETECTION ASSAY (TDA): A VERSATILE PROCEDURE TO DETERMINE DNA BINDING SITES AS DEMONSTRATED ON SP1 PROTEIN" NUCLEIC ACIDS RESEARCH, OXFORD UNIVERSITY PRESS, SURREY, GB, Bd. 18, Nr. 11, 11. Juni 1990 (1990-06-11), Seiten 3203-3209, XP000132496 ISSN: 0305-1048
- FAMULOK M ET AL: "IN VITRO SELECTION OF SPECIFIC LIGAND-BINDING NUCLEIC ACIDS**" ANGEWANDTE CHEMIE. INTERNATIONAL EDITION, VERLAG CHEMIE. WEINHEIM, DE, Bd. 31, Nr. 8, 1. August 1992 (1992-08-01), Seiten 979-988, XP000298700 ISSN: 0570-0833
- PIERROU S ET AL: "SELECTION OF HIGH-AFFINITY BINDING SITES FOR SEQUENCE-SPECIFIC, DNABINDING PROTEINS FROM RANDOM SEQUENCE OLIGONUCLEOTIDES" ANALYTICAL BIOCHEMISTRY, ACADEMIC PRESS, SAN DIEGO, CA, US, Bd. 229, Nr. 1, 20. Juli 1995 (1995-07-20), Seiten 99-105, XP000524716 ISSN: 0003-2697

## Beschreibung

### Gebiet der Erfindung

Die Erfindung betrifft ein Verfahren zur Selektion hochaffin an ein Target bindender Nukleinsäuren, wobei ein Gemisch von Nukleinsäuren mit einem oder mehreren definierten Targetmolekülen kontaktiert wird und wobei an dem Targetmolekül bindende Nukleinsäuren von nicht bindenden Nukleinsäuren getrennt werden.

Als Nukleinsäuren sind Poly- oder Oligonukleotide mit einer Nukleotidzahl von 5 bis 200, insbesondere 20 bis 200, bezeichnet. Es kann sich dabei um DNA, RNA oder PNA handeln. Insbesondere können die Nukleinsäuren chemisch derivatisiert, beispielsweise durch 2' und/oder 5 Substitution, und/oder mit Reportermolekülen (Moleküle, welche einen Nachweis mit üblichen Detektionsmethoden erlauben) versehen sein. Die Nukleinsäure kann einzel- oder doppelsträngig sein. Ein Targetmolekül kann grundsätzlich beliebiger Art sein, sofern es sich nicht seinerseits um eine Nukleinsäure handelt, welche mit der der damit kontaktierten Nukleinsäure Crick/Watson Basenpaar-Bindungen eingeht. Beispiele für Targetmoleküle sind: Kunststoffe, Keramiken, Peptide, Proteine, Enzyme, Oligosacharide, Polysaccharide, Nukleinsäuren, welche nicht-Crick/Watson Bindungen eingehen, Lipide aber auch Hormone und andere organische Verbindungen, wie Pheromone. Targets können auch Zellbestandteile und ganze Zellen sein, ebenso wie vollständige Viren und Bakterien. An nicht-Nukleinsäuren bindende Nukleinsäuren werden auch als Aptamere bezeichnet, aber auch Nukleinsäuren, welche mit anderen Nukleinsäuren nicht-Watson/Crick Bindungen eingehen. Der Begriff der Bindung meint im Rahmen der Erfindung nicht-kovalente Bindungen. Letztgenannte Aptamere können beispielsweise eingesetzt werden, zur Erkennung von bestimmten Gendefekten und/oder Deletionen. Der Begriff der Bindung meint im Rahmen der Erfindung nicht-kovalente Bindungen. Der Begriff der Affinität bezieht sich im Rahmen der Erfindung auf die Bindungsfestigkeit innerhalb von Komplexen der Antigen/Antikörper Art. Die Bindungsfestigkeit ist durch die Affinitätskonstante quantifizierbar, welche definiert ist nach dem Massenwirkungsgesetz. Im Rahmen der Erfindung bezieht sich der Begriff der Affinität allerdings nicht nur auf Komplexe mit einer Bindungsstelle, sondern auch auf Komplexe mit mehreren Bindungsstellen, i.e. umfaßt auch den Begriff der Avidität. Die Avidität berechnet sich aus der Anzahl und der Bindungsstärke jeder Bindungsstelle bei mehrvalenten Antikörper/Antigen Komplexen. Als Amplifikation wird jede enzymatisch vermittelte Reaktion verstanden, die der Replikation einer Nukleinsäuresequenz dient, beispielsweise die PCR.

### Hintergrund der Erfindung und Stand der Technik

Nukleinsäuren dienen in natürlichen Organismen in erster Linie zur Codierung von in einer Zelle zu exprimierenden Proteinen und dergleichen. Hierfür ist die Primärstruktur der Nukleinsäuren, i.e. die Sequenz maßgeblich. Unabhängig hiervon können Nukleinsäuren jedoch auch Antikörper/Antigen Komplexe mit in einer Zelle vorliegenden nicht-Nukleinsäuren eingehen. Ob eine solche Bindung stattfinden kann, hängt dabei nicht nur von der Primärstruktur ab, sondern auch von der in einer Lösung entstehenden Sekundär- und Tertiarstruktur bei einer definierten Sequenz (dreidimensionale Struktur) ab. Die Affinität der Nukleinsäure zu dem Targetmolekül hängt daher letztendlich davon ab, ob die Nukleinsäure - neben der rein chemischen Bindungsfähigkeit - in sterischer Hinsicht im Bereich der Bindungsstelle oder der Bindungsstellen des Targetmoleküls "paßt", entsprechend der Verhältnisse bei klassischen Antikörper/Antigen Komplexen. Passende Nukleinsäuren können also die Funktion eines Antikörpers oder Antigens ausüben. Solche Aptamere sind in aller Regel nicht-natürliche Nukleinsäuren und können gleichsam "maßgeschneidert" werden für ein Targetmolekül. Für die Maßschneiderung gibt es grundsätzlich zwei Ansätze. Der erste Ansatz besteht in der Berechnung einer geeigneten Sequenz und/oder Derivatisierung für die Nukleinsäure nach Maßgabe der genau bekannten Struktur, einschließlich Bindungsstellen und Tertiärstruktur, des Targetmoleküls. Dies ist nicht nur extrem aufwendig; in Fällen, in welchen die Struktur des Targetmoleküls nicht hinreichend bekannt ist, ist dieser Ansatz sogar unmöglich. Der zweite Ansatz besteht in der Isolierung des Targetmoleküls und in der Kontaktierung einer Mischung aus prospektiven Nukleinsäuren mit dem isolierten (und meist immobilisierten) Targetmolekül, wobei hochaffin bindende Nukleinsäuren von den weniger affin oder gar nicht bindenden Nukleinsäuren abgetrennt werden. Bei den Mischungen mit Nukleinsäuren handelt es sich typischerweise um Nukleinsäurenbibliotheken, welche beispielsweise im Wege der kombinatorischen Chemie hergestellt wurden. Eine Nukleinsäurebibliothek enthält eine Vielzahl voneinander unterschiedlicher Nukleinsäuren, wobei zumindest in einem Teilsequenzbereich eine Randomisierung (mit natürlichen und/oder nicht-natürlichen Nukleotiden) eingerichtet ist. Es kann, muß aber nicht, ein konservierter Sequenzbereich vorgesehen sein. Randomisierung in n Positionen mit m verschiedenen Nukleotiden führt zu einer Biblithek mit n^{m} Elementen. Die selektierten hochaffinen Nukleinsäuren sind für eine Vielzahl von Anwendungen geeignet.

So können die Nukleinsäuren im Rahmen von Tests auf Vorliegen oder Nichtvorliegen der für die Nukleinsäure spezifischen Targetmoleküle in einer Testlösung und/oder in einer Zelle bzw. Gewebe verwendet werden. Dann empfiehlt sich die Anwesenheit eines Reportermoleküls üblichen Aufbaus in der Nukleinsäure zur leichten meßtechnischen Identifizierbarkeit. Solche Tests können in der Diagnostik, beispielsweise der Diagnostik auf onkogene Mutanten oder Markersubstanzen, welche bei bestimmten pysiologischen Fehlfunktionen entstehen, eingesetzt werden. Es versteht sich, daß die betreffende Nukleinsäure dann nicht nur die onkogene Mutante selektiv "erkennen" muß, sondern an die natürliche Variante demgegenüber nicht binden darf, i.e. diskriminieren muß zwischen onkogenem Expressionsprodukt und natürlichem Expressionsprodukt. Dies läßt sich leicht nach der Selektion an das onkogene Expressionprodukt bindender Nukleinsäuren durchführen, nämlich durch die subsequente Selektion von an das natürliche Expressionsprodukt nicht bindender Nukleinsäuren aus den zuvor selektierten Nukleinsäuren.

Selektierte Nukleinsäuren können auch zur Abtrennung von Targetmolekülen aus einer Lösung verwendet werden, beispielsweise im Rahmen üblicher Säulen- oder Geltrennungsverfahren. Dann empfiehlt es sich, die Nukleinsäure immobilisierbar zu gestalten und in dem Trennungsverfahren zu immobilisieren.

Selektierte Nukleinsäuren können aber auch dazu eingesetzt werden, um physiologische Funktionen zu modulieren, i.e. zu hemmen, zu induzieren oder zu verstärken. Solche Nukleinsäuren haben daher auch Verwendung in pharmazeutischen Zusammensetzungen. Selektierte Nukleinsäuren müssen natürlich ansonsten physiologisch unbedenklich sein zur Vermeidung von Nebenwirkungen. Der Vorteil des Einsatzes von Nukleinsäuren anstelle von beispielsweise Peptiden liegt darin, daß die Identifizierung bzw. Selektion geeigneter Nukleinsäuren erheblich einfacher als in Falle der Peptide und Proteine ist wegen der vergleichsweise einfachen Herstellbarkeit von Nukleinsäurebibliotheken gegenüber Protein- oder Peptidbibliotheken.

Ein bekanntes Verfahren zur Selektion hochaffin an ein Targetmolekül bindender Nukleinsäuren ist als das SE-LEX Verfahren (Systematic Evolution of Ligands by EXponential Enrichment) bekannt. Verschiedene Varianten und Anwendungsbereich sind beispielsweise beschrieben in den Literaturstellen US-A-5,712,375, US-A-5,864,026, US-A-5,789,157, US-A-5,475,096, US-A-5,861,254, US-A-5,595,877, US-A-5,817,785. Bei dem insofern bekannten Verfahren werden grundsätzlich approximativ in einer Mehrzahl von Zyklen diejenigen Nukleinsäuren separiert, welche mit hoher bzw. immer höherer Affinität binden. Dabei muß im Rahmem jedes Zyklus die selektierte Gruppe an Nukleinsäuren amplifiziert werden. Die Loslösung bindender Nukleinsäuren vom Target in jedem Zyklus erfolgt durch spezifische Verdrängung. Dieses Verfahren hat mehrere Nachteile. Zunächst ist nachteilig, daß aufgrund der erforderlichen Zyklenzahl eine recht große Menge sowohl an Nukleinsäuren als auch an Targetmolekülen erforderlich ist. Weiterhin werden bei der Verdrängungsverfahrenstufe mehr (gebundene) Nukleinsäuren geringerer Affinität aus der Bindung verdrängt als Nukleinsäuren höherer Affinität, wodurch der Mengenunterschied zu Lasten der-höheraffinen Nukleinsäuren im Rahmen der Amplifikationsverfahrensstufe verstärkt wird. Der Mengenunterschied wird zudem noch weiter dadurch verstärkt, daß bei höherer Affinität die Bindung der losgelösten Nukleinsäuren an die zur Loslösung eingesetzten Liganden vergleichsweise stärker ist und die höheraffinen Nukleinsäuren dadurch schlechter für die Amplifikation zugänglich sind. Nachteilig ist weiterhin, daß mit zunehmender Affinität der Nukleinsäuren eine logarithmisch ansteigende Konzentration des zur Loslösung verwendeten Liganden erforderlich wird. Die erreichbare Affinität ist somit vom Löslichkeitsprodukt der eingesetzten Liganden limitiert. Schließlich ist es nachteilig, in mehreren Zyklen arbeiten zu müssen zur wiederholten Abtrennung bzw. Selektion in einem Vorzyklus selektierter Nukleinsäuren.

Im Bereich der Trennung von nicht-Nukleinsäuren ist die Affinitätschromatographie, insbesondere als Säulenchromatographie (Fest-/Flüssigphase) wohl bekannt. Es handelt sich hierbei um ein Verfahren zur Isolierung oder Anreicherung, bis zu 10⁵-fach und mehr, von beispielsweise Proteinen. Ein Ligand der anzureichernden Substanz wird dabei an einer chromatographischen Matrix immobilisiert. Hochaffine Verbindungen werden zuerst, i.e. säuleneingangsseitig, gebunden. Stromabwärts werden weniger affine Verbindungen gebunden, sofern die Menge der affinen Verbindungen, bezogen auf jeweilige konkrete Verbindung, niedriger als die Menge der Liganden in der Säule ist. Gering affine oder nicht affine Verbindungen laufen durch und werden so von den affinen Verbindungen getrennt. Gebundene, i.e. affine Verbindungen werden dann eluiert und weiter verwendet. Bei unspezifischen Desorptionsverfahren, beispielsweise physikochemischen oder thermischen Verfahren, findet ein Vermengung unterschiedlich hoch affiner (gebundener) Verbindungen statt. Bei der Desorption mit Liganden der gebundenen Verbindungen (Verdrängungsdesorption) ist eine sehr große molare Menge des Liganden zur Loslösung insbesondere der hochaffinen Verbindungen notwendig.

Hinzu kommt generell, daß die Trennleistung dann zu wünschen übrig läßt, wenn die Targetmoleküle klein bzw. sehr klein, beispielsweise 50 bis 10000 Da, insbesondere 70 bis 2000 Da, sind und eine Derivatisierung der Targetmoleküle zur Verbesserung der Trennleistung vermieden werden soll. Die strukturelle Heterogenität der Nukleinsäuren einer kombinatorischen Nukleinsäurebibliothek erschwert oder macht unmöglich in diesem Falle die Abtrennung der Nukleinsäure/ Targetmolekül-Komplexe von den nicht komplexierten Nukleinsäuren der Nukleinsäurebibliothek durch eine einfache lineare bzw. eindimensionale Auftrennung.

Aus den Literaturstellen H.J. Thiesen et al., Nucleic Acids Research 18:3208-3209 (1990) und US-A-5707796 sind Verfahren zur Selektion hochaffin an ein Targetmolekül bindender Nukleinsauren bekannt.

### Technisches Problem der Erfindung

Der Erfindung liegt das technische Problem zugrunde, ein Verfahren zur Selektion hochaffin an ein Targetmolekül bindender Nukleinsäuren anzugeben, welches mit weniger Aufwand hochaffine Nukleinsäuren insbesondere gegen sehr kleine Targetmoleküle liefert, und zwar ohne daß eine Derivatisierung der Targetmoleküle erforderlich ist.

### Grundzüge der Erfindung

Zur Lösung dieses Problems lehrt die Erfindung ein Verfahren gemäß Anspruch 1.

Als physikochemische Verfahren kommen insbesondere in Frage: Elektrophorese (z.B. Kapillarelektrophorese, Flachbettelektrophorese, Horizontalelektrophorese), und die Chromatographieverfahren (z.B. Fest-Flüssig Chromatographie, Flüssig-Flüssig-Chromatographie, Kapillarelektrochromatographie). Die hierfür im einzelnen zu wählenden Detailmethoden und Reagenzien sind nach Maßgabe der Targetmoleküle und/oder der Nukleinsäuren vom Fachmann unschwer feststellbar. In physikochemischen Trennungsverfahren erfolgt eine Trennung der aufgetragenen Substanzgemische in Fraktionen, wobei jeder Fraktion ein Laufparameter bzw. ein Laufparameterfenster zugeordnet ist. Als Laufparameter kommen beispielsweise in Frage Zeit und/oder Weg. Ein Laufparameterfenster bezeichnet einen Anfangswert und einen Endwert des Laufparameters, innerhalb dessen eine Fraktion gewonnen wird. Eine Fraktion kann eine oder mehrere Nukleinsäurespezies enthalten. Ein Bindungsmix bezeichnet eine Mischung aus verschiedenen Nukleinsäure/Targetmolekül-Komplexen. Der Begriff der zweidimensionalen Auftrennung bezieht sich auf die subsequente Anwendung des gleichen Trennungsverfahrens einerseits auf die Nukleinsäuren und andererseits der Komplexe. Dies wird bildlich auch deutlich im Rahmen der Ausführungsbeispiele.

Es kann eine einzige Spezies an Targetmolekülen eingesetzt werden, es können aber auch mehrere verschiedene definierte oder undefinierte Spezies, verwendet werden. Die Lösung der Nukleinsäuren und der Targetmoleküle erfolgt in üblichen Puffern, beispielsweise Tris-Puffer oder Acetat-Puffer. Ein Gemisch aus Nukleinsäuren bezeichnet Nukleinsäurebibliotheken mit einer Anzahl von typischerweise 10⁶ bis 10²²/Mol, insbesondere 10⁶ bis 10²¹/Mol voneinander verschiedener Nukleinsäurenspezies. In einer eingesetzten Bibliothek ist jede Nukleinsäurespezies statistische beispielsweise mit 10 bis 10¹⁷, insbesondere 10 bis ₁₀¹³, Molekülen vertreten. Die Bindung der Nukleinsäuren an die Targetmoleküle erfolgt vorzugsweise unter Bedingungen, welche einem späteren Einsatz der Nukleinsäuren entsprechen, i.e. beispielsweise in einem Puffer, welcher entsprechend abgestimmt ist hinsichtlich Temperatur, Ionenstärke, pH-Wert und Pufferbedingungen. Das Lösungsmittel der Nukleinsäurenbibliothek ist dann entsprechend hinsichtlich dessen Komponenten auszuwählen. Gleiches gilt für das Lösungsmittel, in welchem die Targetmoleküle gelöst sind.

Die Erfindung beruht auf der überraschenden Erkenntnis, daß die Bindung eines Targetmoleküls an eine Nukleinsäure einen Einfluß auf das Verhalten der Nukleinsäure in einem physikochemischen Trennungsverfahren hat. Dieser Einfluß ist umso größer, je größer die Affinitätskonstante der Bindung, i.e. je fester die Bindung zwischen Nukleinsäuren und Targetmolekül ist. Die Erfindung eignet sich insbesondere zur Selektion von Nukleinsäuren gegen unmodifizierte kleine Targetmoleküle, beispielsweise 50 bis 10000 Da, insbesondere 70 bis 2000 Da. Denn eine chemische Modifikation von sehr kleinen Targetmolekülen erschwert oder macht unmöglich eine Selektion von gegen das unmodifizierte Targetmolekül affinen Nukleinsäuren.

Subsequente Selektionsartefakte zu Lasten höheraffiner Nukleinsäuren werden zudem vermieden. Ebensowenig sind Liganden, insbesondere hohe Konzentrationen an Liganden, zur Desorption erforderlich. Schließlich stehen praktisch alle gebundenen und dann desorbierten Nukleinsäuremoleküle für eine Amplifikation zur Verfügung. Dies erlaubt es, mit geringen Nukleinsäurekonzentrationen zu arbeiten. Es ist grundsätzlich bereits ausreichend, wenn in der Nukleinsäurebibliothek jede Spezies im statistischen Mittel mit einem Molekül vertreten ist.

Mit dem erfindungsgemäßen Verfahren isolierte Nukleinsäuren bzw. hergestellte Nukleinsäuregemische (kurz Nukleinsäuren) lassen sich vielfältig verwenden. Für eine jeweilige konkrete Anwendung ist es lediglich notwendig, nach Maßgabe der Anwendung ausgewählte Targetmoleküle in der Stufe b) einzusetzen. So ist es beispielsweise möglich, für eine Erkrankung charakteristische Markersubstanzen zu identifizieren, mit dem erfindungsgemäßen Verfahren hochaffin daran bindende Nukleinsäuren zu bestimmen und die so selektierten Nukleinsäuren als Hauptkomponente eines Testkits zur Untersuchung auf die Markersubstanz bzw. auf das Vorliegen der Erkrankung oder das Risiko der Erkrankung einzusetzen. Solche Testkits können selbstverständlich auch zur Therapiekontrolle eingesetzt werden. Die Nukleinsäuren können auch zur Herstellung von pharmazeutischen Zusammensetzungen verwendet werden, beispielsweise wenn eine Hemmung der Markersubstanz (durch Bindung der Nukleinsäure) zu einer Verringerung oder Verhinderung des Krankheitsbildes führt. Durch Komplexierung kleiner Targetmoleküle durch hochaffine Nukleinsäuren kann zusätzlich eine verbesserte venale Ausscheidung insbesondere von unpolaren Targets erreicht werden. Es ist aber auch möglich, durch Auswahl der Targetmoleküle Nukleinsäuren zu selektieren und als pharmazeutische Zusammensetzung zu verwenden, welche durch Aktivierung geeigneter Targetmoleküle die Bildung von im Krankheitsfall fehlenden Stoffen in einem Organismus stimulieren. Durch Auswahl geeigneter Targetmoleküle lassen sich beispielsweise auch Nukleinsäuren finden, welche als Wirkstoffe die Differenzierung und/oder Stimulation oder Suppression von isolierten Zellen (beispielsweise Blutzellen, wie T-Zellen, Granulozyten, Monozyten oder Thrombozyten) beeinflussen. Gleiches kann für Zellen in einem Verbund (Gewebe) u.a. im Rahmen des Tissue Engineering bewirkt werden. Schließlich können erfindungsgemäß selektierte Nukleinsäuren beispielsweise in einer Affinitätsmatrix als immobilisierte Phase eingesetzt werden (z.B. Apherese) oder zur spezifischen Desorption von Substanzen von einer Affinitätsmatrix verwendet werden.

Ein wesentliches Element der Erfindung ist die Selektion an sehr kleinen Targetmolekülen bindender Nukleinsäuren. Denn diese können die Targetmoleküle weitreichend umhüllen und daurch die Elimination der Targetmoleküle z.B. aus der Blutzirkulation verhindern.

### Bevorzugte Ausführungsformen der Erfindung

Anschließend an Stufe c) wird meist eine Trennungsverfahrenstufe d) durchgeführt werden, in welcher die nukleinsäuren selektierter Nukleinsäure/Targetmolekül-Komplexe von den Targetmolekülen getrennt werden. Nach Stufe b), ggf. nach Dissoziation in Stufe c) der erhaltenen Nukleinsäure/Targetmolekül-Komplexe kann eine Amplifikation mittels beispielsweise PCR, RT-PCT oder LCR, durchgeführt werden.

Eine Dissoziation (in einer Trennungsverfahrensstufe) mit dem erfindungsgemäßen Verfahren gewonnener Nukleinsäure/Targetmolekül-Komplexe kann beispielsweise durch Verdrängung mit einem hinreichend starken Liganden, Modifikation, Komplexierung und/oder Zerstörung des Targetmoleküls, physikochemisch oder thermisch erfolgen. Auch mechanische Verfahren, beispielsweise Ultraschall, können zur Dissoziation oder zur Verstärkung der Dissoziation eingesetzt werden. Es versteht sich, daß die Nukleinsäuren von dem verwendeten Dissoziationsverfahren nicht zersetzt werden dürfen. Vorzugsweise wird die unspezifische Dissoziation mittels üblicher physikochemischer oder thermischer Verfahren durchgeführt. Thermische Dissoziation erfolgt durch Erwärmung der erhaltenen Lösung. Die Erwärmung kann beispielsweise durch elektrische Beheizung oder Einstrahlung von Mikrowellen oder IR erfolgen. Insbesondere sind die Erwärmungstechniken aus der PCR Technologie geeignet.

Die unspezifische Dissoziation kann durch chemische Modifikation des Targets unterstützt werden, z.B. Oxidation durch Natriumperjodat oder dergleichen, oder durch unspezifische Komplexbildung, z.B. mittels Borat oder dergleichen zur Blockierung von cis-trans-Diolbindungen in Kohlenhydraten. Es ist besonders bevorzugt, wenn die unspezifische Dissoziation durch thermische Dissoziation in einer, vorzugsweise verlängerten, Hochtemperaturphase einer PCR oder RT-PCR durchgeführt wird. Hierbei wird ein Synergieeffekt erzielt, da in aller Regel, insbesondere beim Arbeiten mit Nukleinsäurebibliotheken mit niedrigen Konzentrationen der Nukleinsäurespezies, eine Amplifizierung ohnehin erforderlich ist. Die (nach der Dissoziation) gewonnenen Nukleinsäuren lassen sich leicht und ohne störende Ligandenkoppelungen amplifizieren.

Zur Erhöhung der Ausbeute wird bei der Amplifikation, PCR, RT-PCR, LCR oder dergleichen, beispielsweise mit 5 bis 60, vorzugsweise mit 20 bis 60, höchstvorzugsweise mit 45 bis 55, Zyklen gearbeitet. Im Rahmen der Amplifikation kann mit mindestens einem markierten Primer gearbeitet werden. Der Primer kann mindestens eine Endonukleaseschnittstelle aufweisen. Eine solche Schnittstelle dient beispielsweise dazu, das Amplifikat von größeren Bereichen der Primersequenz zu befreien. Nukleotidbausteine, sei es im Primer oder bei den zu selektierenden Nukleinsäuren, können beispielsweise mittels Fluoreszenzfarbstoffen markiert sein. Als Fluoreszenzfarbstoffe seien beispielsweise genannt: Alexa³ Fluor 488, Fluor 532, Fluor 546, Fluor 568, Fluor 594, Oregon Green 488, Fluorescein, Rhodamine 6G, Tetramethylrhodamine, Rhodamine B und Texas Red. Das Amplifikat kann auch durch zwei verschiedene chemische Modifikationen an verschiedenen Enden markiert sein, sofern die bei der Modifikation eingeführten. Gruppen geeignet sind, als Liganden jeweils an einer unterschiedlichen Affinitätsmatrix gebunden werden zu können.

Bevorzugt ist es, wenn die Nukleinsäuren für das physikochemische Trennungsverfahren funktionalisiert sind. Wenn als physikochemisches Verfahren beispielsweise ein elektrophoretisches Verfahren eingesetzt wird, so können die Nukleinsäuren (der diversen Spezies) mit einer eine elektrische Ladung tragenden Gruppe ausgestattet sein.

Im Folgenden wird die Erfindung anhand von nicht limitierenden Ausführungsbeispielen näher erläutert.

### Beispiel 1: Selektion durch Laufweg-Trennmethoden

Eine auf übliche Weise erstellte Nukleinsäurebibliothek wird auf ein geeignetes Gel, z.B. Agarosegel, aufgetragen. Nach Erwärmung zum Aufschmelzen eventuell doppelsträngiger Nukleinsäuren wird bei 4°C elektrophoretisch getrennt, und zwar in einer Raumdimension, dem Laufweg. Dann wird das Gel in Streifen in Richtung parallel zur Laufrichtung geschnitten und die Streifen werden mit Targetmolekülen inkubiert. Ein so erhaltener Streifen mit Komplexen wird auf ein gleichartiges zweites Agarosegel gebracht und mittels mechanischer und/oder physikochemischer Methoden werden die Komplexe des Streifens auf das zweite Gel übertragen. Dann wird unter gleichen Bedingungen nochmals elektrophoretisch getrennt, wobei die Laufrichtung orthogonal zur Längserstreckung des aufgebrachten Streifens liegt, i.e. in einer zweiten Raumdimension. Nukleinsäuren, welche keine Komplexe gebildet haben, liegen dann in dem zweiten Gel auf einer Diagonalen. Das zweite Gel wird in Streifen geschnitten, deren Längserstreckung orthogonal zu jener des aufgebrachten Streifens ist. Aus einem so erhaltenen Streifen des zweiten Gels wird jener Bereich herausgetrennt, der dem Laufwegfenster des dem Streifen zugeordneten Teiles des aufgebrachten Streifens entspricht. Der Rest des Streifens wird gesammelt und einer Dissoziationsverfahrensstufe sowie einer Amplifikationsverfahrensstufe unterworfen.

### Beispiel 2: Selektion durch Laufzeit-Trennmethoden.

Eine auf übliche Weise erstellte Nukleinsäurebibliothek wird auf eine geeignete HPLC aufgetragen. Dann wird eine Trennung der Nukleinsäuren in einer Zeitdimension, der Laufzeit, durchgeführt, wobei in definierten Laufzeitfenstern Fraktionen aufgefangen werden. Eine solche Fraktion wird dann mit den Targetmolekülen auf geeignete Weise inkubiert. Die inkubierten Fraktionen werden jeweils sodann unter gleichen Bedingungen nochmals getrennt, i.e: in einer zweiten Zeitdimension. Von den so erhaltenen Fraktionen der zweiten HPLC werden jene Fraktionen verworfen, deren Laufzeit der Laufzeit der aufgegebenen Fraktion entspricht. Die verbleibenden Fraktionen der zweiten HPLC werden gesammelt und einer Dissoziationsverfahrensstufe sowie einer Amplifikationsverfahrensstufe unterworfen. Entsprechendes wird für alle Fraktionen der ersten HPLC durchgeführt.

## Patentansprüche

1. Verfahren zur Selektion hochaffin an ein Targetmolekül bindender Nukleinsäuren aus einem Gemisch von Nukleinsäuren mit den folgenden Verfahrensschritten:
a) das Gemisch aus Nukleinsäuren wird einem physikochemischen Trennungsverfahren unterworfen, wobei ein Set von Nukleinsäuregemischfraktionen erhalten wird, und wobei jeder Nukleinsäuregemischfraktion ein Laufparameterfenster zugeordnet wird,
b) die Nukleinsäuregemischfraktionen werden jeweils mit den Targetmolekülen kontaktiert, wobei Bindungsmixe mit Nukleinsäure/Targetmolekül-Komplexen erhalten werden,
c) die Bindungsmixe aus Stufe b) werden jeweils dem gleichen physikochemischen Trennungsverfahren wie in Stufe a) unterworfen, wobei Nukleinsäure/Targetmolekül-Komplexe, deren Laufparameter außerhalb des jeweiligen Laufparameterfensters liegen, selektiert werden.

2. Verfahren nach Anspruch 1, wobei anschließend an Stufe c) eine Trennungsverfahrenstufe d) durchgeführt wird, in welcher die Nukleinsäuren selektierter Nukleinsäure/Targetmolekül-Komplexe von den Targetmolekülen getrennt werden.

3. Verfahren nach Anspruch 1 oder 2, wobei die selektierten Nukleinsäuren amplifiziert werden.

## Claims

1. A method of selection of nucleic acids that bind to a target molecule with high affinity from a mixture of nucleic acids, comprising the following steps:
a) the mixture of nucleic acids is subjected to,a physicochemical separation method, wherein a set of mixed fractions containing the nucleic acids is obtained, and wherein a run parameter window is associated with every mixed fraction containing the nucleic acids,
b) the mixed fractions containing the nucleic acids are each contacted with the target molecules, whereby binding mixtures containing nucleic acid/target molecule complexes are obtained,
c) the binding mixtures from step b) are each subjected to the same physicochemical separation step as in step a), whereby nucleic acid/target molecule complexes whose run parameters are outside the respective run parameter window, are selected.

2. The method according to claim 1, wherein subsequently to step c) a separation step d) is carried out, in which the nucleic acids of selected nucleic acid/target molecule complexes are separated from the target molecules.

3. The method according to claim 1 or 2, wherein the selected nucleic acids are amplified.

## Revendications

1. Procédé de sélection d'acides nucléiques, qui se lient à une molécule cible avec une haute affinité, à partir d'un mélange d'acides nucléiques, comprenant les étapes suivantes:
a) le mélange d'acides nucléiques est soumis à un procédé de séparation physico-chimique, dans lequel un jeu de fractions mélangées comprenant les acides nucléiques est obtenu, et dans lequel une fenêtre de paramètres courants est associée à chaque fraction mélangée comprenant les acides nucléiques,
b) les fractions mélangées comprenant les acides nucléiques sont contactées avec les molécules cibles, par cela des mélanges de liage comprenant des complexes d'acides nucléiques/ molécules cibles étant obtenus,
c) les mélanges de liage à partir de l'étape b) sont chacun soumis à la même étape de séparation physico-chimique comme dans l'étape a), par cela des complexes d'acides nucléiques/molécules cibles, dont les paramètres courants se trouvent à l'extérieur de la fenêtre de paramètres courants, étant sélectionnés.

2. Procédé selon la revendication 1, dans lequel après l'étape c) une étape de séparation d) est effectuée, dans laquelle les acides nucléiques de complexes d'acides nucléiques/molécules cibles sélectionnés sont séparés des molécules cibles.

3. Procédé selon la revendication 1 ou 2, dans lequel les acides nucléiques sélectionnés sont amplifiés.
